(19) Europäisches Patentamt
European Patent Office
Office européen des brevets

(11) Veröffentlichungsnummer: **0 224 140 A1**

(12) # EUROPÄISCHE PATENTANMELDUNG

(21) Anmeldenummer: 86115789.9

(22) Anmeldetag: 13.11.86

(51) Int. Cl.⁴: C 07 D 333/38
C 07 D 333/50, A 01 N 43/10
A 01 N 43/12

(30) Priorität: 26.11.85 DE 3541628

(43) Veröffentlichungstag der Anmeldung: 03.06.87 Patentblatt 87/23

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Hallenbach, Werner, Dr.
Kleiststrasse 10
D-4018 Langenfeld(DE)

(72) Erfinder: Lindel, Hans, Dr.
Carl-Duisberg-Strasse 321
D-5090 Leverkusen 1(DE)

(72) Erfinder: Brandes, Wilhelm, Dr.
Eichendorffstrasse 3
D-5653 Leichlingen(DE)

(54) **Thienylharnstoff-Derivate.**

(57) Thienylharnstoff-Derivate der Formel (I)

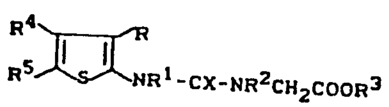

(I)

in welcher
X und R bis $R^5$ die in der Beschreibung angegebenen Bedeutungen haben, und deren Säureadditions-Salze, sowie deren Verwendung als Schädlingsbekämpfungsmittel im Pflanzenschutz.

Die Verbindungen der Formel (I) können hergestellt werden, indem man z.B. geeignete Thienylamine mit geeigneten Iso(thio)cyanaten umsetzt oder geeignete Thienyliso(thio)cyante mit geeigneten Aminosäuren bzw. deren Säureadditionssalzen gegebenenfalls in Gegenwart eines Katalysators und gegebenenfalls in Gegenwart von Verdünnungsmitteln umsetzt und gegebenenfalls physiologisch verträgliche Säuren addiert.

EP 0 224 140 A1

Croydon Printing Company Ltd

BAYER AKTIENGESELLSCHAFT
Konzernverwaltung RP
Patentabteilung

5090 Leverkusen, Bayerwerk

Bas/by-c
Ib

## Thienylharnstoff-Derivate

Die Erfindung betrifft neue Thienylharnstoff-Derivate, mehrere Verfahren zu ihrer Herstellung und ihre Verwendung in Schädlingsbekämpfungsmitteln, insbesondere als Fungizide, im Pflanzenschutz.

Es ist bereits bekannt, daß bestimmte Thienylharnstoffe wie z. B. 1-(4,5-Dimethyl-3-ethoxycarbonyl-2-thienyl)-3-methylharnstoff eine gute fungizide Wirksamkeit besitzen (vergl. z. B. US-PS 3 823 161). Die Wirkung dieser bekannten Verbindungen ist jedoch, insbesondere bei niedrigen Wirkstoffkonzentrationen und Aufwandmengen nicht immer voll zufriedenstellend.

Es wurden nun neue Thienylharnstoff-Derivate der Formel (I)

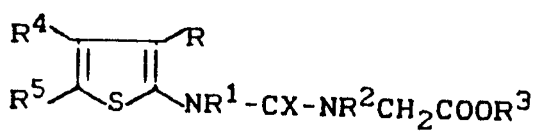

(I)

in welcher

X für Sauerstoff oder Schwefel steht,

R für Cyano, Hydroxycarbonyl oder Alkoxycarbonyl steht,

$R^1$ für Wasserstoff oder Alkyl steht,

$R^2$ für Wasserstoff oder Alkyl steht,

$R^3$ für Wasserstoff, Alkyl oder gegebenenfalls substituiertes Aryl steht,

$R^4$ für Wasserstoff, Alkyl oder gegebenenfalls substituiertes Aryl steht und

$R^5$ für Wasserstoff, Alkyl oder gegebenenfalls substituiertes Aryl steht oder

$R^4$ und $R^5$ gemeinsam für einen Rest $-(CH_2)_n-$ stehen, worin

n für die Zahlen 3, 4, 5 oder 6 steht,

und deren Säureadditions-Salze gefunden.

Weiterhin wurde gefunden, daß man die neuen Thienylharnstoff-Derivate der Formel (I)

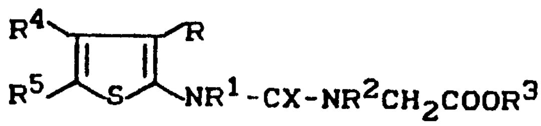

(I)

in welcher

X für Sauerstoff oder Schwefel steht,

R für Cyano, Hydroxycarbonyl oder Alkoxycarbonyl steht,

$R^1$ für Wasserstoff oder Alkyl steht,

$R^2$ für Wasserstoff oder Alkyl steht,

$R^3$ für Wasserstoff, Alkyl oder gegebenenfalls substituiertes Aryl steht,

$R^4$ für Wasserstoff, Alkyl oder gegebenenfalls substituiertes Aryl steht und

$R^5$ für Wasserstoff, Alkyl oder gegebenenfalls substituiertes Aryl steht, oder

$R^4$ und $R^5$ gemeinsam für einen Rest $-(CH_2)_n-$ stehen, worin

n für die Zahlen 3, 4, 5 oder 6 steht,

und deren Säureadditions-Salze erhält, wenn man

) für den Fall, daß $R^2$ für Wasserstoff steht, Thienylamine der Formel (II)

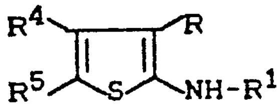

(II) .

in welcher

$R$, $R^1$, $R^4$ und $R^5$ die oben angegebenen Bedeutungen haben,

mit Iso(thio)cyanaten der Formel (III)

$$XCNCH_2COOR^3 \qquad (III)$$

in welcher

X und $R^3$ die oben angegebenen Bedeutungen haben,

gegebenenfalls in Gegenwart von Katalysatoren und gegebenenfalls in Gegenwart von Verdünnungsmitteln umsetzt,

oder

b) für den Fall, daß $R^1$ für Wasserstoff steht, Thienyliso(thio)cyanate der Formel (IV)

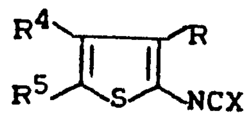

(IV)

in welcher

X, R, $R^4$ und $R^5$ die oben angegebenen Bedeutungen haben,

mit Aminosäuren bzw. deren Säureadditionssalzen der Formel (V)

$$HR^2N-CH_2COOR^3 \qquad (V)$$

in welcher

$R^2$ und $R^3$ die oben angegebenen Bedeutungen haben,

gegebenenfalls in Gegenwart von Katalysatoren und gegebenenfalls in Gegenwart von Verdünnungsmitteln umsetzt und gegebenenfalls an die erhaltenen Verbindungen physiologisch verträgliche Säuren addiert.

Die neuen Thienylharnstoff-Derivate der Formel (I) zeichnen sich durch hohe Wirksamkeit als Schädlingsbekämpfungsmittel, insbesondere durch ihre hervorragende selektive fungizide Wirkung aus.

Die Alkylreste $R^1$, $R^2$, $R^3$, $R^4$ und $R^5$ sowie der Alkylteil des Alkoxycarbonylrestes R können verzweigt oder unverzweigt sein und enthalten vorzugsweise jeweils 1 bis 8, insbesondere 1 bis 6 und besonders bevorzugt 1 bis 4 Kohlenstoffatome. Beispielhaft seien genannt: Methyl, Ethyl,

n-Propyl, iso-Propyl, n-Butyl, iso-Butyl, sec.-Butyl, tert.-Butyl, Methoxycarbonyl, Ethoxycarbonyl, n-Propoxycarbonyl, iso-Propoxycarbonyl, n-Butoxycarbonyl, iso-Butoxycarbonyl, sec.-Butoxycarbonyl und tert.-Butoxycarbonyl.

Die gegebenenfalls substituierten Arylreste $R^3$, $R^4$ und $R^5$ enthalten im Arylteil vorzugsweise 6 bis 10 Kohlenstoffatome. Beispielhaft seien genannt: gegebenenfalls substituiertes Naphthyl oder Phenyl, insbesondere gegebenenfalls substituiertes Phenyl.

Die gegebenenfalls substituierten Arylreste $R^3$, $R^4$ und $R^5$ können einen oder mehrere, vorzugsweise 1 bis 3, insbesondere 1 oder 2 gleiche oder verschiedene Substituenten tragen.

Als Substituenten seien beispielhaft aufgeführt: Halogen wie Fluor, Chlor und Brom; Cyano; Nitro; Phenyl; Phenoxy; Phenylthio; Alkyl mit vorzugsweise 1 bis 4 , insbesondere 1 oder 2 Kohlenstoffatomen wie Methyl, Ethyl, n- und i-Propyl und n-, i-, sec.- und t-Butyl; Alkoxy mit vorzugsweise 1 bis 4, insbesondere 1 oder 2 Kohlenstoffatomen wie Methoxy, Ethoxy, n- und i-Propyloxy und n-, i-, sec.- und t-Butyloxy; Alkylthio mit vorzugsweise 1 bis 4, insbesondere 1 oder 2 Kohlenstoffatomen wie Methylthio, Ethylthio, n- und i-Propylthio und n-, i-, sec.- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor,

Chlor oder Brom, insbesondere Fluor, stehen wie Trifluormethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor oder Brom, insbesondere Fluor stehen wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor oder Brom, insbesondere Fluor, stehen wie Trifluormethylthio; Alkylcarbonyl mit vorzugsweise 1 bis 4, insbesondere 1 oder 2 Kohlenstoffatomen wie Methylcarbonyl und Ethylcarbonyl; Amino; Alkylamino und Dialkylamino mit jeweils vorzugsweise 1 bis 4, insbesondere 1 oder 2 Kohlenstoffatomen wie Methylamino, Ethylamino, n-Propylamino, i-Propylamino, n-Butylamino, i-Butylamino, Dimethylamino, Diethylamino, Di-n-propylamino, Di-i-propylamino, Di-n-butylamino und Di-i-Butylamino; Alkoxyalkyl mit jeweils vorzugsweise 1 bis 4, insbesondere 1 oder 2 Kohlenstoffatomen im Alkylteil-, bzw Alkoxyteil, wie Methoxymethyl, Ethoxymethyl, Methoxyethyl und Ethoxyethyl; gegebenenfalls halogensubstituiertes Methylendioxy oder Ethylendioxy mit gegebenenfalls jeweils 1 bis 4 Halogenatomen, wobei als Halogenatome vorzugsweise Fluor, Chlor oder Brom, insbesondere Fluor und/oder Chlor stehen.

Die Erfindung betrifft vorzugsweise Verbindungen der Formel (I), in welcher

X für Sauerstoff oder Schwefel steht,

R für Cyano, Hydroxycarbonyl oder Alkoxycarbonyl mit 1 bis 6 Kohlenstoffatomen im Alkylteil steht,

$R^1$ für Wasserstoff oder Alkyl mit 1 bis 4 Kohlenstoffatomen steht,

$R^2$ für Wasserstoff oder Alkyl mit 1 bis 4 Kohlenstoffatomen steht,

$R^3$ für Wasserstoff, Alkyl mit 1 bis 6 Kohlenstoffatomen oder für gegebenenfalls einfach bis dreifach, gleich oder verschieden durch Fluor; Chlor; Brom; $C_1$-$C_2$-Alkyl; $C_1$-$C_2$-Alkoxy; $C_1$-$C_2$-Alkylthio; Halogen-$C_1$-$C_2$-alkyl, Halogen-$C_1$-$C_2$-alkoxy und Halogen-$C_1$-$C_2$-alkylthio mit jeweils 1 bis 3 Halogenatomen wie Fluor, Chlor und/oder Brom, insbesondere Fluor und/oder Chlor; Cyano; Nitro; Phenyl; Phenoxy; Phenylthio; $C_1$-$C_2$-Alkoxy-carbonyl; Amino; $C_1$-$C_2$-Alkylamino, Di-($C_1$-$C_2$)-alkylamino; $C_1$-$C_2$-Alkoxy-$C_1$-$C_2$-alkyl sowie gegebenenfalls durch Fluor und/oder Chlor substituiertes Methylendioxy oder Ethylendioxy substituiertes Phenyl steht,

$R^4$ für Wasserstoff, Alkyl mit 1 bis 6 Kohlenstoffatomen oder für gegebenenfalls einfach bis fünffach, gleich oder verschieden substituiertes Phenyl steht, wobei als Phenylsubstituenten vorzugsweise die bei $R^3$ bereits genannten Phenylsubstituenten in Frage kommen und

$R^5$ für Wasserstoff, Alkyl mit 1 bis 6 Kohlenstoffatomen, oder gegebenenfalls ein- bis dreifach, gleich oder verschieden substituiertes Phenyl steht, wobei als Phenylsubstituenten vorzugsweise die bei $R^3$ bereits genannten Phenylsubstituenten in Frage kommen,

oder

$R^4$ und $R^5$ gemeinsam für einen Rest $-(CH_2)_n-$ stehen, worin

n für die Zahlen 3, 4, 5 oder 6 steht.

Besonders bevorzugt sind die Verbindungen der Formel (I), in welcher

X für Sauerstoff oder Schwefel steht,

R für Cyano, Hydroxycarbonyl oder $C_1$-$C_4$-Alkoxycarbonyl steht,

$R^1$ für Wasserstoff oder $C_1$-$C_2$-Alkyl steht,

$R^2$ für Wasserstoff oder $C_1$-$C_2$-Alkyl steht,

$R^3$ für Wasserstoff, $C_1$-$C_4$-Alkyl oder Phenyl steht,

$R^4$ für Wasserstoff, $C_1$-$C_4$-Alkyl oder Phenyl steht und

$R^5$ für Wasserstoff, $C_1$-$C_4$-Alkyl oder Phenyl steht,

oder

$R^4$ und $R^5$ gemeinsam für einen Rest $-(CH_2)_n-$ stehen, worin

n für die Zahlen 3, 4, 5 oder 6 steht.

Ganz besonders bevorzugt sind die Verbindungen der Formel (I), in welcher

X für Sauerstoff steht,

R für Cyano, Hydroxycarbonyl, Methoxycarbonyl, Ethoxycarbonyl, n-Propoxycarbonyl, i-Propoxycarbonyl, n-Butoxycarbonyl, i-Butoxycarbonyl, sec.-Butoxycarbonyl oder tert.-Butoxycarbonyl steht,

$R^1$ für Wasserstoff steht,

$R^2$ für Wasserstoff steht,

$R^3$ für Wasserstoff, Methyl, Ethyl, n-Propyl, i-Propyl, n-Butyl, i-Butyl, sec.-Butyl oder tert.-Butyl steht,

$R^4$ für Wasserstoff, Methyl, Ethyl, n-Propyl, i-Propyl, n-Butyl, i-Butyl, sec.-Butyl oder tert.-Butyl steht und

$R^5$ für Wasserstoff, Phenyl, Methyl, Ethyl, n-Propyl, i-Propyl, n-Butyl, i-Butyl, sec.-Butyl oder tert.-Butyl steht,

oder

$R^4$ und $R^5$ gemeinsam für einen Rest $-(CH_2)_n-$ stehen, worin

n für die Zahlen 3, 4, 5 oder 6 steht.

Weiterhin sind ganz besonders bevorzugt die Verbindungen der Formel (I), in welcher

X für Schwefel steht,

R für Hydroxycarbonyl, Methoxycarbonyl, Ethoxycarbonyl, n-Propoxycarbonyl, i-Propoxycarbonyl, n-Butoxycarbonyl, i-Butoxycarbonyl, sec.-Butoxycarbonyl oder tert.-Butoxycarbonyl steht,

$R^1$ für Wasserstoff steht,

$R^2$ für Wasserstoff steht,

$R^3$ für Wasserstoff, Methyl, Ethyl, n-Propyl, i-Propyl, n-Butyl, i-Butyl, sec.-Butyl oder tert.-Butyl steht,

$R^4$ für Wasserstoff, Methyl, Ethyl, n-Propyl, i-Propyl, n-Butyl, i-Butyl, sec.-Butyl oder tert.-Butyl steht und

$R^5$ für Wasserstoff, Methyl, Ethyl, n-Propyl, i-Propyl, n-Butyl, i-Butyl, sec.-Butyl oder tert.-Butyl steht,

oder

$R^4$ und $R^5$ gemeinsam für einen Rest $-(CH_2)_n-$ stehen, worin

n für die Zahlen 3, 4, 5 oder 6 steht.

Bevorzugte erfindungsgemäße Verbindungen sind auch Additionsprodukte aus Säuren und denjenigen Thienylharnstoff-Derivaten der Formel (I), in denen die Substituenten R, $R^1$, $R^2$, $R^3$, $R^4$ und $R^5$ die Bedeutungen haben, die bereits vorzugsweise für diese Substituenten genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Schwefelsäure, Salpetersäure, Essigsäure, Oxalsäure, Malonsäure, Bernsteinsäure, Äpfelsäure, Weinsäure, Maleinsäure, Fumarsäure, Methansulfonsäure, Benzoesäure, substituierte Benzoesäuren, Ameisensäure, Chloressigsäure, Toluolsulfonsäure, Benzolsulfonsäure, Trichloressigsäure, Phthalsäure, Naphthalinsulfonsäure, Nikotinsäure, Citronensäure und Ascorbinsäure.

Verwendet man beispielsweise für das erfindungsgemäße Verfahren (a) 3-Methoxycarbonyl-4-methyl-2-methylaminothiophen und Isocyanatoessigsäuremethylester als Ausgangsstoffe, so kann die Reaktion durch das folgende Formelschema skizziert werden:

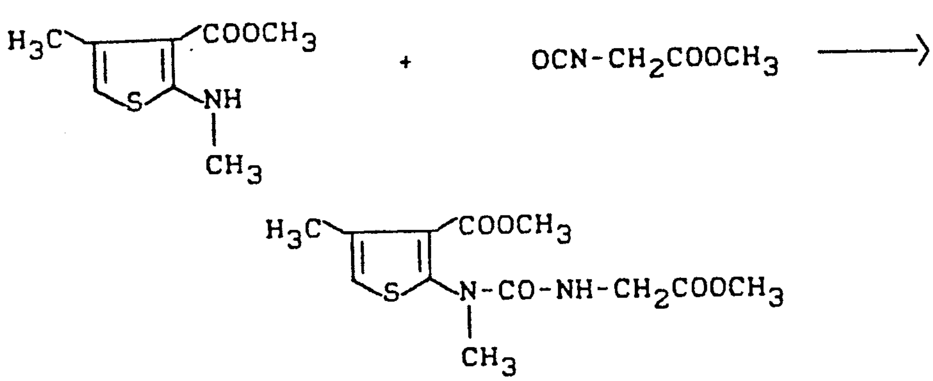

Verwendet man beispielsweise für das erfindungsgemäße Verfahren (b) 2-Isocyanato-4-methyl-3-methoxycarbonyl-thiophen und Glycinmethylester als Ausgangsstoffe, so kann die Reaktion durch das folgende Formelschema skizziert werden:

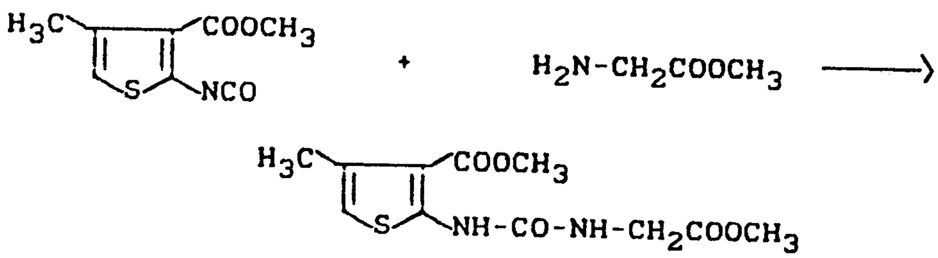

Die für das erfindungsgemäße Verfahren (a) als Ausgangsstoffe einzusetzenden Thienylamine sind durch die Formel (II) definiert. In dieser Formel stehen R, $R^1$, $R^4$ und $R^5$ vorzugsweise für diejenigen Reste, welche bei der Definition in Formel (I) genannt wurden.

Die Verbindungen der Formel (II) sind bekannt oder lassen sich analog zu bekannten Verfahren herstellen (vergl. z. B. K. Gewald Chem. Ber. 98 (1965), S. 3571, Chem. Ber. 99 (1966), S. 94. EP-OS 4 931, G. Coppola et al. J. Heterocycl. Chem. 1982, S. 717).

Im einzelnen seien folgende Verbindungen der Formel (II) genannt:

2-Amino-3-cyano-4,5-trimethylen-thiophen
2-Amino-3-methoxycarbonyl-4,5-trimethylen-thiophen
2-Amino-3-ethoxycarbonyl-4,5-trimethylen-thiophen
2-Amino-3-t-butoxycarbonyl-4,5-trimethylen-thiophen

2-Amino-3-cyano-4,5-tetramethylen-thiophen
2-Amino-3-methoxycarbonyl-4,5-tetramethylen-thiophen
2-Amino-3-ethoxycarbonyl-4,5-tetramethylen-thiophen
2-Amino-3-t-butoxycarbonyl-4,5-tetramethylen-thiophen
2-Amino-3-cyano-4,5-pentamethylen-thiophen
2-Amino-3-methoxycarbonyl-4,5-pentamethylen-thiophen
2-Amino-3-ethoxycarbonyl-4,5-pentamethylen-thiophen
2-Amino-3-t-butoxycarbonyl-4,5-pentamethylen-thiophen
2-Amino-3-hydroxycarbonyl-thiophen
2-Amino-3-hydroxycarbonyl-4-methyl-thiophen
2-Amino-3-hydroxycarbonyl-5-methyl-thiophen
2-Amino-3-hydroxycarbonyl-4,5-dimethyl-thiophen
2-Amino-3-hydroxycarbonyl-4-ethyl-thiophen
2-Amino-3-hydroxycarbonyl-5-ethyl-thiophen
2-Amino-3-hydroxycarbonyl-4,5-diethyl-thiophen
2-Amino-3-hydroxycarbonyl-4-ethyl-5-methyl-thiophen
2-Amino-3-hydroxycarbonyl-4-methyl-5-ethyl-thiophen
2-Amino-3-hydroxycarbonyl-4-i-propyl-thiophen
2-Amino-3-hydroxycarbonyl-5-i-propyl-thiophen
2-Amino-3-hydroxycarbonyl-4-methyl-5-phenyl-thiophen
2-Amino-3-hydroxycarbonyl-4-ethyl-5-phenyl-thiophen
2-Amino-3-hydroxycarbonyl-5-phenyl-thiophen
2-Amino-3-methoxycarbonyl-thiophen
2-Amino-3-methoxycarbonyl-4-methyl-thiophen
2-Amino-3-methoxycarbonyl-5-methyl-thiophen
2-Amino-3-methoxycarbonyl-4,5-dimethyl-thiophen
2-Amino-3-methoxycarbonyl-4-ethyl-thiophen
2-Amino-3-methoxycarbonyl-5-ethyl-thiophen
2-Amino-3-methoxycarbonyl-4,5-diethyl-thiophen
2-Amino-3-methoxycarbonyl-4-ethyl-5-methyl-thiophen
2-Amino-3-methoxycarbonyl-4-methyl-5-ethyl-thiophen
2-Amino-3-methoxycarbonyl-4-i-propyl-thiophen

2-Amino-3-methoxycarbonyl-5-i-propyl-thiophen
2-Amino-3-ethoxycarbonyl-thiophen
2-Amino-3-ethoxycarbonyl-4-methyl-thiophen
2-Amino-3-ethoxycarbonyl-5-methyl-thiophen
2-Amino-3-ethoxycarbonyl-4,5-dimethyl-thiophen
2-Amino-3-ethoxycarbonyl-4-ethyl-thiophen
2-Amino-3-ethoxycarbonyl-5-ethyl-thiophen
2-Amino-3-ethoxycarbonyl-4,5-diethyl-thiophen
2-Amino-3-ethoxycarbonyl-4-ethyl-5-methyl-thiophen
2-Amino-3-ethoxycarbonyl-4-methyl-5-ethyl-thiophen
2-Amino-3-ethoxycarbonyl-4-i-propyl-thiophen
2-Amino-3-ethoxycarbonyl-5-i-propyl-thiophen
2-Amino-3-methoxycarbonyl-4-methyl-5-phenyl-thiophen
2-Amino-3-methoxycarbonyl-4-ethyl-5-phenyl-thiophen
2-Amino-3-methoxycarbonyl-5-phenyl-thiophen
2-Amino-3-ethoxycarbonyl-4-methyl-5-phenyl-thiophen
2-Amino-3-ethoxycarbonyl-4-ethyl-5-phenyl-thiophen
2-Amino-3-ethoxycarbonyl-5-phenyl-thiophen
2-Amino-3-cyano-thiophen
2-Amino-3-cyano-4-methyl-thiophen
2-Amino-3-cyano-5-methyl-thiophen
2-Amino-3-cyano-4,5-dimethyl-thiophen
2-Amino-3-cyano-4-ethyl-thiophen
2-Amino-3-cyano-5-ethyl-thiophen
2-Amino-3-cyano-4,5-diethyl-thiophen
2-Amino-3-cyano-4-ethyl-5-methyl-thiophen
2-Amino-3-cyano-4-methyl-5-ethyl-thiophen
2-Amino-3-cyano-4-i-propyl-thiophen
2-Amino-3-cyano-5-i-propyl-thiophen
2-Amino-3-cyano-4-methyl-5-phenyl-thiophen
2-Amino-3-cyano-4-ethyl-5-phenyl-thiophen

2-Amino-3-cyano-5-phenyl-thiophen und die entsprechenden 2-Methylamino-Derivate.

Die ebenfalls als Ausgangsstoffe für das erfindungsgemäße Verfahren (a) zu verwendenden Iso(thio)cyanate sind durch die Formel (III) definiert. In dieser Formel stehen X und $R^3$ vorzugsweise für diejenigen Reste, welche bei der Definition in Formel (I) genannt wurden.

Die Verbindungen der Formel (III) sind bekannt (vergl. z. B. "Methoden der organischen Chemie" (Houben-Weyl-Müller), Band XV/2, S. 183, Thieme Verlag - Stuttgart).

Als Beispiele für die Verbindungen der Formel (III) seien genannt:
Isocyanato-essigsäuremethylester, Isocyanato-essigsäureethylester, Isocyanato-essigsäure-n-propylester, Isocyanato-essigsäure-i-propylester, Isocyanato-essigsäure-n-butylester, Isocyanato-essigsäure-i-butylester, Isocyanato-essigsäure-sec.-butylester, Isocyanato-essigsäure-tert.-butylester, Isocyanato-essigsäurephenylester und die entsprechenden Isothiocyanato-Derivate.

Die erfindungsgemäße Umsetzung (a) zwischen den Thienylaminen der Formel (II) und den Iso(thio)cyanaten der Formel (III) führt man vorzugsweise in Gegenwart eines Verdünnungsmittels durch. Als solche eignen sich alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylester, Tetrahydrofuran und Dioxan, weiterhin Ketone wie Aceton, Methylethyl-, Methylisopropyl- und Methylisobutylketon, außerdem Ester, wie Essigsäure-methylester und -ethylester, ferner Nitrile wie z. B. Acetonitril und Propionitril, Benzonitril, Glutarsäuredinitril, darüber hinaus Amide wie z. B. Dimethylformamid, Dimethylacetamid und N-Methylpyrrolidon sowie Dimethylsulfoxid, Tetramethylensulfon und Hexamethylphosphorsäuretriamid.

Zur Beschleunigung des erfindungsgemäßen Verfahrens (a) können Katalysatoren zugesetzt werden. Als solche sind geeignet: z. B. tertiäre Amine wie Pyridin, 4-Dimethylaminopyridin, Triethylamin, Triethylendiamin, Trimethylen-tetrahydropyrimidin; ferner Zinn-II- und Zinn-IV-Verbindungen wie Zinn-II-octoat oder Zinn-IV- chlorid. Die als Reaktionsbeschleuniger genannten tertiären Amine, z. B. Pyridin, können auch als Lösungsmittel verwendet werden.

Die Reaktionstemperaturen des erfindungsgemäßen Verfahrens (a) können in einem größeren Temperaturbereich variiert werden. Im allgemeinen arbeitet man zwischen 0 °C und 120 °C, vorzugsweise zwischen 20 ° und 70 °C.

Normalerweise arbeitet man unter Normaldruck, jedoch kann es zweckmäßig sein, z.B. beim Einsatz niedrig siedender Iso(thio)cyanate, in geschlossenen Gefäßen unter Druck zu arbeiten.

Bei der Durchführung des erfindungsgemäßen Verfahrens (a) setzt man die Ausgangsstoffe der Formeln (II) und (III) im allgemeinen in stöchiometrischen Verhältnissen ein, günstig ist jedoch ein geringer Überschuß des Isocyanats. Die Katalysatoren werden vorzugsweise in Mengen von 0,01 bis 0,1 Mol pro Mol der Reaktionskomponenten angewandt, jedoch sind auch größere Mengen, z. B. der tertiären Amine, anwendbar. Die Reaktionsprodukte werden isoliert, indem man aus den entsprechenden Lösungsmitteln direkt ausfallende Produkte filtriert oder indem man das Lösungsmittel abdestilliert.

Die für das erfindungsgemäße Verfahren (b) als Ausgangsstoffe einzusetzenden Thienyliso(thio)cyanate sind durch die Formel (IV) definiert. In dieser Formel stehen X, R, $R^4$ und $R^5$ vorzugsweise für diejenigen Reste, welche bei der Definition in Formel (I) genannt wurden.

Die Verbindungen der Formel (IV) sind bekannt und/oder lassen sich nach bekannten Verfahren herstellen (vergl. z. B. DE-AS 20 40 579, DE-AS 21 22 636, EP-OS 4 931 und eine eigene nicht vorveröffentlichte Anmeldung die Deutsche Patentanmeldung P 35 29 247).

Als Beispiele für die Verbindungen der Formel (IV) seien genannt:

2-Isocyanato-3-cyano-4,5-trimethylen-thiophen
2-Isocyanato-3-methoxycarbonyl-4,5-trimethylen-thiophen
2-Isocyanato-3-ethoxycarbonyl-4,5-trimethylen-thiophen
2-Isocyanato-3-t-butoxycarbonyl-4,5-trimethylen-thiophen

2-Isocyanato-3-cyano-4,5-tetramethylen-thiophen
2-Isocyanato-3-methoxycarbonyl-4,5-tetramethylen-thiophen
2-Isocyanato-3-ethoxycarbonyl-4,5-tetramethylen-thiophen
2-Isocyanato-3-t-butoxycarbonyl-4,5-tetramethylen-thiophen
2-Isocyanato-3-cyano-4,5-pentamethylen-thiophen
2-Isocyanato-3-methoxycarbonyl-4,5-pentamethylen-thiophen
2-Isocyanato-3-ethoxycarbonyl-4,5-pentamethylen-thiophen
2-Isocyanato-3-t-butoxycarbonyl-4,5-pentamethylen-thiophen
2-Isocyanato-3-methoxycarbonyl-thiophen
2-Isocyanato-3-methoxycarbonyl-4-methyl-thiophen
2-Isocyanato-3-methoxycarbonyl-5-methyl-thiophen
2-Isocyanato-3-methoxycarbonyl-4,5-dimethyl-thiophen
2-Isocyanato-3-methoxycarbonyl-4-ethyl-thiophen
2-Isocyanato-3-methoxycarbonyl-5-ethyl-thiophen
2-Isocyanato-3-methoxycarbonyl-4,5-diethyl-thiophen
2-Isocyanato-3-methoxycarbonyl-4-ethyl-5-methyl-thiophen
2-Isocyanato-3-methoxycarbonyl-4-methyl-5-ethyl-thiophen
2-Isocyanato-3-methoxycarbonyl-4-i-propyl-thiophen
2-Isocyanato-3-methoxycarbonyl-5-i-propyl-thiophen
2-Isocyanato-3-ethoxycarbonyl-thiophen
2-Isocyanato-3-ethoxycarbonyl-4-methyl-thiophen
2-Isocyanato-3-ethoxycarbonyl-5-methyl-thiophen
2-Isocyanato-3-ethoxycarbonyl-4,5-dimethyl-thiophen
2-Isocyanato-3-ethoxycarbonyl-4-ethyl-thiophen
2-Isocyanato-3-ethoxycarbonyl-5-ethyl-thiophen
2-Isocyanato-3-ethoxycarbonyl-4,5-diethyl-thiophen
2-Isocyanato-3-ethoxycarbonyl-4-ethyl-5-methyl-thiophen
2-Isocyanato-3-ethoxycarbonyl-4-methyl-5-ethyl-thiophen
2-Isocyanato-3-ethoxycarbonyl-4-i-propyl-thiophen

0224140

2-Isocyanato-3-ethoxycarbonyl-5-i-propyl-thiophen
2-Isocyanato-3-methoxycarbonyl-4-methyl-5-phenyl-thiophen
2-Isocyanato-3-methoxycarbonyl-4-ethyl-5-phenyl-thiophen
2-Isocyanato-3-methoxycarbonyl-5-phenyl-thiophen
2-Isocyanato-3-ethoxycarbonyl-4-methyl-5-phenyl-thiophen
2-Isocyanato-3-ethoxycarbonyl-4-ethyl-5-phenyl-thiophen
2-Isocyanato-3-ethoxycarbonyl-5-phenyl-thiophen
2-Isocyanato-3-cyano-thiophen
2-Isocyanato-3-cyano-4-methyl-thiophen
2-Isocyanato-3-cyano-5-methyl-thiophen
2-Isocyanato-3-cyano-4,5-dimethyl-thiophen
2-Isocyanato-3-cyano-4-ethyl-thiophen
2-Isocyanato-3-cyano-5-ethyl-thiophen
2-Isocyanato-3-cyano-4,5-diethyl-thiophen
2-Isocyanato-3-cyano-4-ethyl-5-methyl-thiophen
2-Isocyanato-3-cyano-4-methyl-5-ethyl-thiophen
2-Isocyanato-3-cyano-4-i-propyl-thiophen
2-Isocyanato-3-cyano-5-i-propyl-thiophen
2-Isocyanato-3-cyano-4-methyl-5-phenyl-thiophen
2-Isocyanato-3-cyano-4-ethyl-5-phenyl-thiophen
2-Isocyanato-3-cyano-5-phenyl-thiophen und die entsprechenden 2-Isothiocyanato-Derivate.

Die ebenfalls als Ausgangsstoffe für das Verfahren (b) zu verwendenden Aminosäuren sind durch die Formel (V) definiert. In dieser Formel stehen $R^2$ und $R^3$ vorzugsweise für diejenigen Reste, welche bei der Definition in Formel (I) genannt wurden. Als Ausgangsstoffe für das Verfahren (b) können auch die Säureadditions-Salze der Aminosäuren der Formel (V) eingesetzt werden. Zu den Säuren, die addiert werden können, gehören vorzugsweise die Säuren, die bereits für die Säureadditions-Salze der Verbindungen der Formel (I) genannt wurden.

Die Verbindungen der Formel (V) sind allgemein bekannte Verbindungen der organischen Chemie.

Als Beispiele für die Verbindungen der Formel (V) bzw. deren entsprechenden Säureadditions-Salze seien genannt:

$$HR^2N-CH_2COOR^3 \qquad (V)$$

Tabelle 1

| $R^2$ | $R^3$ | $R^2$ | $R^3$ |
|---|---|---|---|
| H | $CH_3$ | $CH_3$ | $CH_3$ |
| H | $C_2H_5$ | $CH_3$ | $C_2H_5$ |
| H | $C_3H_7$-n | $CH_3$ | $C_3H_7$-n |
| H | $C_3H_7$-i | $CH_3$ | $C_3H_7$-i |
| H | $C_4H_9$-n | $CH_3$ | $C_4H_9$-n |
| H | $C_4H_9$-i | $CH_3$ | $C_4H_9$-i |
| H | $C_4H_9$-s | $CH_3$ | $C_4H_9$-s |
| H | $C_4H_9$-t | $CH_3$ | $C_4H_9$-t |
| H | H | $CH_3$ | H |
| H | 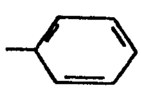 | $CH_3$ | 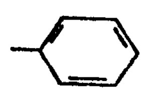 |

Das erfindungsgemäße Verfahren (b) kann mit oder ohne Verdünnungsmittel durchgeführt werden. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, weiterhin Ketone wie Aceton, Methylethyl-, Methylisopropyl- und Methylisobutylketon, außerdem Ester wie Essigsäure-methylester und -ethylester, ferner Nitrile wie z. B. Acetonitril und Propionitril, Benzonitril, Glutarsäuredinitril, darüber hinaus Amide wie z. B. Dimethylformamid, Dimethylacetamid und N-Methylpyrrolidon sowie Dimethylsulfoxid, Tetramethylensulfon und Hexamethylphosphorsäuretriamid.

Zur Beschleunigung des erfindungsgemäßen Verfahrens (b) können Katalysatoren zugesetzt werden. Als solche sind geeignet: z. B. tertiäre Amine wie Pyridin, 4-Dimethylaminopyridin, Triethylamin, Triethylendiamin, Trimethylen-tetrahydropyrimidin; ferner Zinn-II- und Zinn-IV-Verbindungen wie Zinn-II-octoat oder Zinn-IV- chlorid. Die als Reaktionsbeschleuniger genannten tertiären Amine, z. B. Pyridin, können auch als Lösungsmittel verwendet werden.

Die Reaktionstemperaturen können in einem größeren Temperaturbereich variiert werden. Im allgemeinen arbeitet man zwischen 0 °C und 120 °C, vorzugsweise zwischen 20 °C und

70 °C.

Normalerweise arbeitet man unter Normaldruck, jedoch kann es zweckmäßig sein, z. B. beim Einsatz niedrig siedender Amine, in geschlossenen Gefäßen unter Druck zu arbeiten.

Bei der Durchführung des erfindungsgemäßen Verfahrens (b) setzt man die Ausgangsstoffe der Formeln (IV) und (V) im allgemeinen in stöchiometrischen Verhältnissen ein, günstig ist jedoch ein geringer Überschuß des Amins. Die Katalysatoren werden vorzugsweise in Mengen von 0,01 bis 0,1 Mol pro Mol der Reaktionskomponenten angewandt, jedoch sind auch größere Mengen, z. B. der tertiären Amine, anwendbar.

Die Reaktionsprodukte werden isoliert, indem man aus den entsprechenden Lösungsmitteln direkt ausfallende Produkte filtriert oder indem man das Lösungsmittel abdestilliert.

Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäßen Wirkstoffe weisen eine starke mikrobizide Wirkung auf und können zur Bekämpfung von unerwünschten Mikroorganismen praktisch eingesetzt werden.

Die Wirkstoffe sind für den Gebrauch als Schädlingsbekämpfungsmittel, vor allem als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;

Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielseise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die neuen Wirkstoffe entfalten eine besonders gute Wirksamkeit gegen Botrytispilze, z. B. gegen Botrytis cinerea, den Erreger des Grauschimmels an Gartenbohnen, Salat, Erdbeeren und Reben.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie

Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beispiel A

Botrytis-Test (Rebe)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test Verbindungen z.B. gemäß den Herstellungsbeispielen: (6) und (5)

## Herstellungsbeispiele

### Beispiel 1

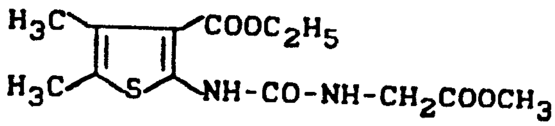

(Verfahren (a))

Zu 5 g (0,22 Mol) 2-Amino-4,5-dimethyl-3-ethoxycarbonyl-thiophen (vergl. dazu Chem. Ber. 99, S. 94 - 100 (1966)) in 30 ml trockenem Pyridin werden 4 g (0,35 Mol) Isocyanato-essigsäuremethylester gegeben und 4 Stunden auf 70 °C erwärmt. Nach Abkühlung wird in überschüssige verdünnte Salzsäure eingerührt, der Niederschlag abfiltriert und aus Ethanol umkristallisiert.

Man erhält 2,6 g (37,3 % der Theorie) 1-(4,5-Dimethyl-3-ethoxycarbonyl-2-thienyl)-3-methoxycarbonylmethyl-harnstoff vom Schmelzpunkt 151 °C.

### Beispiel 2

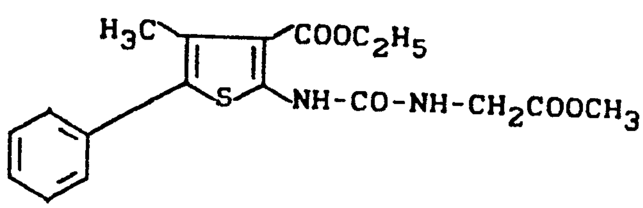

4,26 g (0,034 Mol) Glycinmethylester-hydrochlorid und 3,43 g (0,034 Mol) Triethylamin werden in 40 ml trockenem Chloroform vorgelegt und 6,5 g (0,023 Mol) 3-Ethoxycarbonyl-2-isocyanato-4-methyl-5-phenyl-thiophen gelöst in 20 ml trockenem Chloroform zugetropft. Es wird noch 30 Minuten nachgerührt, zur Aufarbeitung wird auf 300 ml Wasser gegossen, die organische Phase abgetrennt und das Lösungsmittel im Vakuum abdestilliert. Der Rückstand wird über Kieselgel/Methylenchlorid chromatographiert.

Man erhält 5,3 g (62,3 % der Theorie) 1-(3-Ethoxycarbonyl-4-methyl-5-phenyl-2-thienyl)-3-methoxycarbonylmethyl-harnstoff.

IR (Film) : 3400, 3000, 1740, 1660, 1550, 1520, 1220, 1050 und 1020 $cm^{-1}$.

Analog Beispiel (1) und (2) bzw. Verfahren (a) und (b) können die nachfolgenden Verbindungen der Formel (I) hergestellt werden:

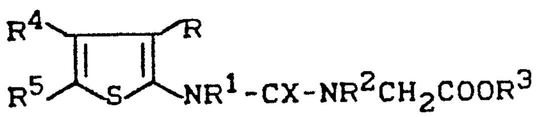 (I)

Tabelle 2

| Bsp.-Nr. | R | $R^1$ | $R^2$ | $R^3$ | $R^4$ | $R^5$ | X | Schmelzpunkt [°C] |
|---|---|---|---|---|---|---|---|---|
| 3 | $-COOC_2H_5$ | H | H | $-CH_3$ | H | H | O | 146 |
| 4 | $-COOC_2H_5$ | H | H | $-CH_3$ | $-C_2H_5$ | $-CH_3$ | O | 131 |
| 5 | $-COOH$ | H | H | $-CH_3$ | H | $-CH_3$ | O | 202 |
| 6 | $-COOC_2H_5$ | H | H | $-CH_3$ | $-(CH_2)_4-$ | | O | 166 |
| 7 | $-COOC_2H_5$ | H | H | $-CH_3$ | $-(CH_2)_5-$ | | O | 125 |
| 8 | $-COOC_4H_9$-t | H | H | $-CH_3$ | $-(CH_2)_5-$ | | O | 109 |
| 9 | $-COOCH_3$ | H | H | $-CH_3$ | H | 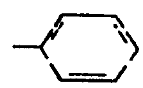 | O | |
| 10 | $-COOC_2H_5$ | H | H | $-CH_3$ | H | $-C_3H_7$-i | O | 119 |
| 11 | $-COOCH_3$ | H | H | $-CH_3$ | $-CH_3$ | H | O | |
| 12 | $-COOC_2H_5$ | H | H | $-CH_3$ | H | $-CH_3$ | O | |
| 13 | $-COOC_2H_5$ | H | H | $-CH_3$ | $-CH_3$ | $-C_2H_5$ | O | |
| 14 | $-COOCH_3$ | H | H | $-CH_3$ | $-(CH_2)_3-$ | | O | 184 |
| 15 | $-COOC_2H_5$ | H | H | $-CH_3$ | $-(CH_2)_3-$ | | O | 173 |

Tabelle 2 - Fortsetzung

| Bsp.-Nr. | R | $R^1$ | $R^2$ | $R^3$ | $R^4$ | $R^5$ | X | Schmelzpunkt [°C] |
|---|---|---|---|---|---|---|---|---|
| 16 | $-COOC_4H_9$-t | H | H | $-CH_3$ | $-(CH_2)_3-$ | | O | 185 |
| 17 | -CN | H | H | $-CH_3$ | $-(CH_2)_3-$ | | O | 168 |
| 18 | $-COOCH_3$ | H | H | $-CH_3$ | $-(CH_2)_4-$ | | O | 146 |
| 19 | $-COOC_4H_9$-t | H | H | $-CH_3$ | $-(CH_2)_4-$ | | O | Öl |
| 20 | -CN | H | H | $-CH_3$ | $-(CH_2)_4-$ | | O | 154 |
| 21 | $-COOCH_3$ | H | H | $-CH_3$ | $-(CH_2)_5-$ | | O | 123 |
| 22 | -CN | H | H | $-CH_3$ | $-(CH_2)_4-$ | | O | 184 |
| 23 | $-COOC_2H_5$ | H | H | $-CH_3$ | H | H | S | |
| 24 | $-COOCH_3$ | H | H | $-CH_3$ | H | 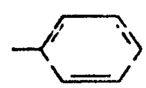 | S | |
| 25 | $-COOC_2H_5$ | H | H | $-CH_3$ | H | $-CH_3$ | S | |
| 26 | -CN | H | H | $-CH_3$ | $-CH_3$ | H | O | 176 |
| 27 | -CN | H | H | $-CH_3$ | H | H | O | 157 |
| 28 | -CN | H | H | $-CH_3$ | H | $-CH_3$ | O | 159 |
| 29 | -CN | H | H | $-C_2H_5$ | $-CH_3$ | $-CH_3$ | O | |

Patentansprüche

1. Thienylharnstoff-Derivate der Formel (I)

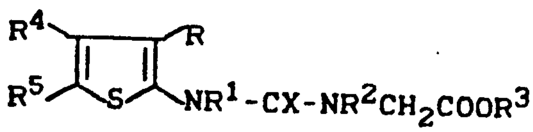

(I)

in welcher

X für Sauerstoff oder Schwefel steht,

R für Cyano, Hydroxycarbonyl oder Alkoxycarbonyl steht,

$R^1$ für Wasserstoff oder Alkyl steht,

$R^2$ für Wasserstoff oder Alkyl steht,

$R^3$ für Wasserstoff, Alkyl oder gegebenenfalls substituiertes Aryl steht,

$R^4$ für Wasserstoff, Alkyl oder gegebenenfalls substituiertes Aryl steht und

$R^5$ für Wasserstoff, Alkyl oder gegebenenfalls substituiertes Aryl steht, oder

$R^4$ und $R^5$ gemeinsam für einen Rest $-(CH_2)_n-$ stehen, worin

n für die Zahlen 3, 4, 5 oder 6 steht,

und deren Säureadditions-Salze.

2. Thienylharnstoff-Derivate der Formel (I) gemäß Anspruch 1, worin

X für Sauerstoff oder Schwefel steht,

R für Cyano, Hydroxycarbonyl oder Alkoxycarbonyl mit 1 bis 6 Kohlenstoffatomen im Alkylteil steht,

$R^1$ für Wasserstoff oder Alkyl mit 1 bis 4 Kohlenstoffatomen steht,

$R^2$ für Wasserstoff oder Alkyl mit 1 bis 4 Kohlenstoffatomen steht,

$R^3$ für Wasserstoff, Alkyl mit 1 bis 6 Kohlenstoffatomen oder für gegebenenfalls einfach bis dreifach, gleich oder verschieden durch Fluor; Chlor; Brom; $C_1$-$C_2$-Alkyl; $C_1$-$C_2$-Alkoxy; $C_1$-$C_2$-Alkylthio; Halogen-$C_1$-$C_2$-alkyl, Halogen-$C_1$-$C_2$-alkoxy und Halogen-$C_1$-$C_2$-alkylthio mit jeweils 1 bis 3 Halogenatomen; Cyano; Nitro; Phenyl; Phenoxy; Phenylthio; $C_1$-$C_2$-Alkoxy-carbonyl; Amino; $C_1$-$C_2$-Alkylamino, Di-($C_1$-$C_2$)-alkylamino; $C_1$-$C_2$-Alkoxy-$C_1$-$C_2$-alkyl und gegebenenfalls durch Fluor und/oder Chlor substituiertes Methylendioxy oder Ethylendioxy substituiertes Phenyl steht,

$R^4$ für Wasserstoff, Alkyl mit 1 bis 6 Kohlenstoffatomen oder für gegebenenfalls einfach bis fünffach, gleich oder verschieden substituiertes Phenyl steht, wobei als Phenylsubstituenten die bei $R^3$ bereits genannten Phenylsubstituenten genannt seien

$R^5$ für Wasserstoff, Alkyl mit 1 bis 6 Kohlenstoffatomen, oder gegebenenfalls ein- bis dreifach, gleich oder verschieden substituiertes Phenyl steht, wobei als Phenylsubstituenten die bei $R^3$ genannten Phenylsubstituenten genannt seien

oder

$R^4$ und $R^5$ gemeinsam für einen Rest $-(CH_2)_n-$ stehen, worin

n für die Zahlen 3, 4, 5 oder 6 steht.

3. Thienylharnstoff-Derivate der Formel (I) gemäß Anspruch 1, worin

X für Sauerstoff oder Schwefel steht,

R für Cyano, Hydroxycarbonyl oder $C_1$-$C_4$-Alkoxycarbonyl steht,

$R^1$ für Wasserstoff oder $C_1$-$C_2$-Alkyl steht,

$R^2$ für Wasserstoff oder $C_1$-$C_2$-Alkyl steht,

$R^3$ für Wasserstoff, $C_1$-$C_4$-Alkyl oder Phenyl steht,

$R^4$ für Wasserstoff, $C_1$-$C_4$-Alkyl oder Phenyl steht und

$R^5$ für Wasserstoff, $C_1$-$C_4$-Alkyl oder Phenyl steht,

oder

$R^4$ und $R^5$ gemeinsam für einen Rest $-(CH_2)_n-$ stehen, worin

n für die Zahlen 3, 4, 5 oder 6 steht.

4. Thienylharnstoff-Derivate der Formel (I) gemäß Anspruch 1, worin

X für Sauerstoff steht,

R für Cyano, Hydroxycarbonyl, Methoxycarbonyl, Ethoxycarbonyl, n-Propoxycarbonyl, i-Propoxycarbonyl, n-Butoxycarbonyl, i-Butoxycarbonyl, sec.-Butoxycarbonyl oder tert.-Butoxycarbonyl steht,

$R^1$ für Wasserstoff steht,

$R^2$ für Wasserstoff steht,

$R^3$ für Wasserstoff, Methyl, Ethyl, n-Propyl, i-Propyl, n-Butyl, i-Butyl, sec.-Butyl oder tert.-Butyl steht,

$R^4$ für Wasserstoff, Methyl, Ethyl, n-Propyl, i-Propyl, n-Butyl, i-Butyl, sec.-Butyl oder tert.-Butyl steht und

$R^5$ für Wasserstoff, Phenyl, Methyl, Ethyl, n-Propyl, i-Propyl, n-Butyl, i-Butyl, sec.-Butyl oder tert.-Butyl steht,

oder

$R^4$ und $R^5$ gemeinsam für einen Rest $-(CH_2)_n-$ stehen, worin

n für die Zahlen 3, 4, 5 oder 6 steht.

5. Thienylharnstoff-Derivate der Formel (I) gemäß Anspruch 1, worin

X für Schwefel steht,

R für Hydroxycarbonyl, Methoxycarbonyl, Ethoxycarbonyl, n-Propoxycarbonyl, i-Propoxycarbonyl, n-Butoxycarbonyl, i-Butoxycarbonyl, sec.-Butoxycarbonyl oder tert.-Butoxycarbonyl steht,

$R^1$ für Wasserstoff steht,

$R^2$ für Wasserstoff steht,

$R^3$ für Wasserstoff, Methyl, Ethyl, n-Propyl, i-Propyl, n-Butyl, i-Butyl, sec.-Butyl oder tert.-Butyl steht,

$R^4$ für Wasserstoff, Methyl, Ethyl, n-Propyl, i-Propyl, n-Butyl, i-Butyl, sec.-Butyl oder tert.-Butyl steht und

$R^5$ für Wasserstoff, Methyl, Ethyl, n-Propyl, i-Propyl, n-Butyl, i-Butyl, sec.-Butyl oder tert.-Butyl steht,

oder

$R^4$ und $R^5$ gemeinsam für einen Rest $-(CH_2)_n-$ stehen, worin

n für die Zahlen 3, 4, 5 oder 6 steht.

6. Verfahren zur Herstellung von Thienylharnstoff-Derivaten der Formel (I)

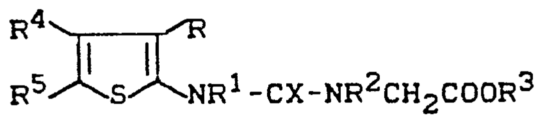 (I)

in welcher

X für Sauerstoff oder Schwefel steht,

R für Cyano, Hydroxycarbonyl oder Alkoxycarbonyl steht,

$R^1$ für Wasserstoff oder Alkyl steht,

$R^2$ für Wasserstoff oder Alkyl steht,

$R^3$ für Wasserstoff, Alkyl oder gegebenenfalls substituiertes Aryl steht,

$R^4$ für Wasserstoff, Alkyl oder gegebenenfalls substituiertes Aryl steht und

$R^5$ für Wasserstoff, Alkyl oder gegebenenfalls substituiertes Aryl steht oder

$R^4$ und $R^5$ gemeinsam für einen Rest $-(CH_2)_n-$ stehen, worin

n für die Zahlen 3, 4, 5 oder 6 steht,

und deren physiologisch verträglichen Säureadditions-Salze, dadurch gekennzeichnet, daß man

a) für den Fall, daß $R^2$ für Wasserstoff steht, Thienylamine der Formel (II)

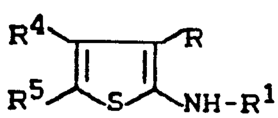

(II)

in welcher

R, $R^1$, $R^4$ und $R^5$ die oben angegebenen Bedeutungen haben,

mit Iso(thio)cyanaten der Formel (III)

$$XCNCH_2COOR^3 \qquad (III)$$

in welcher

X und $R^3$ die oben angegebenen Bedeutungen haben,

gegebenenfalls in Gegenwart von Katalysatoren und gegebenenfalls in Gegenwart von Verdünnungsmitteln umsetzt,

oder

b) für den Fall, daß $R^1$ für Wasserstoff steht, Thienyliso(thio)cyanate der Formel (IV)

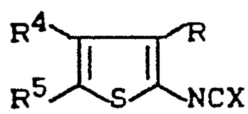

(IV)

in welcher

X, R, $R^4$ und $R^5$ die oben angegebenen Bedeutungen haben,

mit Aminosäuren bzw. deren Säureadditionssalzen der Formel (V)

$$HR^2N\text{-}CH_2COOR^3 \qquad (V)$$

in welcher

$R^2$ und $R^3$ die oben angegebenen Bedeutungen haben,

gegebenenfalls in Gegenwart von Katalysatoren und gegebenenfalls in Gegenwart von Verdünnungsmitteln umsetzt und gegebenenfalls an die erhaltenen Verbindungen der Formel (I) physiologisch verträgliche Säuren addiert.

7. Schädlingsbekämpfungsmittel, gekennzeichnet durch einen Gehalt an mindestens einem Thienylharnstoff-Derivat der Formel (I) nach den Ansprüchen 1 bis 6.

8. Verwendung von Thienylharnstoff-Derivaten der Formel (I) nach den Ansprüchen 1 bis 6 zur Bekämpfung von Schädlingen.

9. Verfahren zur Bekämpfung von Schädlingen, dadurch gekennzeichnet, daß man Thienylharnstoff-Derivate der Formel (I) nach den Ansprüchen 1 bis 6 auf Schädlinge und/oder ihren Lebensraum einwirken läßt.

10. Verfahren zur Herstellung von Schädlingsbekämpfungsmitteln, dadurch gekennzeichnet, daß man Thienylharnstoff-Derivate der Formel (I) nach den Ansprüchen 1 bis 6 mit Streckmitteln und/oder oberflächenaktiven Mitteln vermischt.

Europäisches Patentamt

# EUROPÄISCHER RECHERCHENBERICHT

Nummer der Anmeldung

EP 86115789.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| X | CHEMICAL ABSTRACTS, Band 99, Nr. 9, 29 August 1983, Columbus, Ohio, USA<br>F.KIENZLE et al. "Synthesis of 2,3, 4,5-1H-tetrahydro imidazo[2,1-b]-quinazoline-2,5-diones and analogous 2,3,4,5-1H-tetra-hydroimidazo [1,2-a]thieno [2,3-d] (or[3,2-d])-pyrimidine-2,5-diones"<br>Seite 623, Spalte 1, Zusammenfassung-Nr. 70 661j<br>& Helv. Chim. Acta 1983, 66(1), 148-57<br>-- | 1-3,5, 6 | C 07 D 333/38<br>C 07 D 333/50<br>A 01 N 43/10<br>A 01 N 43/12 |
| A | GB - A - 1 323 553 (MAY & BAKER)<br>* Ansprüche *<br>-- | 1,6-10 | |
| A | GB - A - 1 462 570 (CHEVRON RESEARCH)<br>* Gesamt; insbesondere Ansprüche *<br>---- | 1,6-10 | **RECHERCHIERTE SACHGEBIETE (Int Cl 4)**<br>C 07 D 333/00<br>A 01 N 43/00 |

Der vorliegende Recherchenbericht wurde für alle Patentansprüche erstellt.

| Recherchenort | Abschlußdatum der Recherche | Prüfer |
|---|---|---|
| WIEN | 05-02-1987 | HOFBAUER |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument